# EUROPEAN PATENT APPLICATION

(11) **EP 3 885 430 A1**
(43) Date of publication of application: **29.09.2021**
(21) Application number: 19901649.4
(22) Date of filing: 26.12.2019
(51) Int. Cl.: C12N 1/20, C12N 1/21, C12R 1/19

(54) **MEDIUM FOR INDUCING PLASMID COPY NUMBER INCREASE AND APPLICATION THEREOF**

(30) Priority: 27.12.2018 CN 201811610822
(71) Applicant: Jiangsu Genscript Biotech Co., Ltd., Jiangsu 212000 (CN)
(72) Inventor: ZHAO, Fanglong, Nanjing, Jiangsu 211100 (CN); MA, Yanqiu, Zhenjiang New District, Jiangsu 212000 (CN); WANG, Man, Zhenjiang New District, Jiangsu 212000 (CN); LIU, Fan, Zhenjiang New District, Jiangsu 212000 (CN); WU, Cheng-Hsien, Zhenjiang New District, Jiangsu 212000 (CN)
(74) Representative: Wang, Bo
(86) International application number: PCT/CN2019/128523
(87) International publication number: WO 2020/135549

(57) **Abstract**

The present invention provides a culture medium for inducing an increase in a plasmid copy number and use thereof. The culture medium for inducing an increase in a plasmid copy number of the present invention has a plasmid extraction concentration increased by from 45 to 95% compared with a conventional method for inducing a plasmid copy number, and has a plasmid extraction concentration increased by from 110 to 440% compared with an induction method using a culture medium without glucose and arabinose. The culture medium plays an important role in inducing an increase in a plasmid copy number and achieving high-throughput production.

## Description

This application claims priority to Chinese Patent Application No. 201811610822.6, entitled "CULTURE MEDIUM FOR INDUCING INCREASE OF PLASMID COPY NUMBER AND USE THEREOF" filed with the China National Intellectual Property Administration on December 27, 2018, which is incorporated herein by reference in its entirety.

### BACKGROUND

### Technical Field

The present invention relates to the field of biotechnologies, and specifically, to a culture medium for inducing an increase in a plasmid copy number and use thereof.

### Related Art

In the field of gene synthesis, about 10% or more of gene sequences produce toxicities to host *Escherichia coli.* These toxicities are usually introduced by heterologous genes expressed into proteins by the host to affect the physiological metabolism of the host and further the growth of the host. To resist toxic genes, the host *Escherichia coli* tends to retain plasmids with gene mutation or gene deletion, resulting in reduced stability of the genes and plasmids. The use of low-copy plasmids in production can effectively reduce expression of the toxic genes, and improve growth viability of the host and stability of genes. However, the low-copy plasmids have a low yield and are difficult to extract, thereby affecting production efficiency. To resolve the problem, it is necessary to rapidly induce the low-copy plasmids to have a high copy number before plasmid extraction, thereby improving the plasmid yield. However, existing methods for inducing an increase in a plasmid copy number require a plurality of operations such as transfer, OD600 quantification, and addition of an inducer, which are relatively complex and have difficulty in meeting requirements of actual high-throughput production.

### SUMMARY

To resolve the problem of cumbersome operations of an existing plasmid copy number induction system, the present invention provides a culture medium for inducing an increase in a plasmid copy number and use thereof, which improves production efficiency while simplifying operations.

An aspect of the present invention provides a culture medium for inducing an increase in a plasmid copy number, including tryptone, a yeast extract, glucose, sodium chloride, and arabinose.

In some implementations, the glucose has a concentration of from 0.1 to 10 g/L, preferably from 1 to 5 g/L, and more preferably from 0.5 to 2 g/L.

In some implementations, the glucose has a concentration of from 0.1 to 10 g/L. In some implementations, the glucose has a concentration of from 1 to 5 g/L. In some implementations, the glucose has a concentration of from 0.5 to 2 g/L. In some implementations, the glucose has a concentration of 0.5 g/L, 0.6 g/L, 0.7 g/L, 0.8 g/L, 0.9 g/L, 1 g/L, 1.1 g/L, 1.2 g/L, 1.3 g/L, 1.4 g/L, 1.5 g/L, 1.6 g/L, 1.7 g/L, 1.8 g/L, 1.9 g/L, or 2 g/L. In some implementations, the glucose has a concentration of 0.5 g/L. In some implementations, the glucose has a concentration of 1 g/L. In some implementations, the glucose has a concentration of 2 g/L. In some implementations, the glucose has a concentration of 10 g/L.

In some other implementations, the arabinose has a concentration of from 0.1 to 10 g/L, preferably from 1 to 5 g/L, and more preferably from 0.3 to 5 g/L.

In some other implementations, the arabinose has a concentration of from 0.1 to 10 g/L. In some implementations, the arabinose has a concentration of from 1 to 5 g/L. In some implementations, the arabinose has a concentration of from 0.3 to 0.75 g/L. In some implementations, the arabinose has a concentration of 0.3 g/L, 0.35 g/L, 0.4 g/L, 0.45 g/L, 0.5 g/L, 0.55 g/L, 0.6 g/L, 0.65 g/L, 0.7 g/L, or 0.75 g/L. In some implementations, the arabinose has a concentration of 0.3 g/L. In some implementations, the arabinose has a concentration of 0.6 g/L. In some implementations, the arabinose has a concentration of 0.75 g/L. In some implementations, the arabinose has a concentration of 1 g/L. In some implementations, the arabinose has a concentration of 2 g/L. In some implementations, the arabinose has a concentration of 5 g/L.

In some other implementations, the tryptone has a concentration of from 1 to 15 g/L.

In some other preferred implementations, the tryptone has a concentration of from 5 to 10 g/L.

In some other implementations, the yeast extract has a concentration of from 1 to 15 g/L.

In some other preferred implementations, the yeast extract has a concentration of from 5 to 10 g/L.

In some other implementations, the sodium chloride has a concentration of from 1 to 15 g/L.

In some other preferred implementations, the sodium chloride has a concentration of from 5 to 10 g/L.

In some more preferred implementations, the culture medium further includes one or more of magnesium chloride, potassium chloride, ferric chloride, or calcium chloride.

In some implementations, the plasmid is a plasmid including a replication origin oriV.

In some implementations, the plasmid is a single-copy plasmid including a replication origin oriV.

In some implementations, the plasmid is a single-copy pCCIBAC plasmid.

Another aspect of the present invention provides a method for inducing an increase in a plasmid copy number, including the following steps:
Step (1): transforming *Escherichia coli* with a plasmid; and
Step (2): inoculating the *Escherichia coli* obtained in Step (1) into the culture medium for culturing under conditions suitable for culture.

In some implementations, in Step (1), the plasmid is transformed into competent *Escherichia coli.*

In some other implementations, in Step (2), the *Escherichia coli* is preferably EPI300 *Escherichia coli* or EPI400 *Escherichia coli.*

In some other implementations, in Step (2), a culture temperature is from 20 to 37°C.

In some other implementations, in Step (2), a culture temperature is from 25 to 37°C.

In some other implementations, in Step (2), a culture time is from 4 to 6 h.

In some specific implementations, in Step (1), a single-copy plasmid including a replication origin oriV is transformed into an *Escherichia coli* strain, and in Step (2), the Escherichia coli is cultured at a temperature of from 20 to 37°C in a culture medium including glucose with a concentration of from 1 to 5 g/L, arabinose with a concentration of from 1 to 5 g/L, tryptone with a concentration of from 1 to 15 g/L, a yeast extract with a concentration of from 1 to 15 g/L, and sodium chloride with a concentration of from 1 to 15 g/L.

In some other specific implementations, in Step (1), the pCCIBAC plasmid is transformed into EPI300 *Escherichia coli* or EPI400 *Escherichia coli,* and in Step (2), the *Escherichia coli* is cultured at a temperature of from 25 to 37°C in a culture medium including glucose with a concentration of from 0.5 to 2 g/L, arabinose with a concentration of from 0.3 to 0.75 g/L, tryptone with a concentration of from 5 to 10 g/L, a yeast extract with a concentration of from 5 to 10 g/L, and NaCl with a concentration of from 5 to10 g/L.

Still another aspect of the present invention provides use of the culture medium in inducing an increase in a plasmid copy number.

The present invention provides a novel culture medium that can induce an increase in a plasmid copy number and improve the plasmid yield. In addition, when the culture medium is used for inducing an increase in a plasmid copy number, operation steps are simple, a culture time is greatly shortened, and a plurality of operations, such as transfer, OD600 quantification, and addition of an inducer, are not required. When the concentration of glucose is lower than 2 g/L, and the concentration of arabinose is lower than 0.75 g/L, the concentration of plasmid extraction is significantly higher than that of a culture medium with other concentrations of the same components. Especially, when the concentration of glucose is from 0.5 to 2 g/L, and the concentration of arabinose is from 0.3 to 0.75 g/L, compared with a conventional method for inducing a plasmid copy number, the concentration of plasmid extraction is increased by more than 45%, and compared with an induction method using a culture medium without glucose and arabinose, the concentration of plasmid extraction is increased by more than 310%. The culture medium plays an important role in inducing an increase in a plasmid copy number and achieving high-throughput production.

### Explanation of Terms

As used herein, the term "plasmid" refers to a DNA molecule outside a chromosome (or a nucleoid) within organisms such as bacteria, yeasts, and actinomycetes. The plasmid exists in cytoplasm and can replicate independently, so that the plasmid can also maintain a constant copy number in progeny cells and express carried genetic information. The plasmid is a closed circular, double-stranded DNA molecule.

The term "pCCIBAC plasmid" refers to a single-copy bacterial artificial chromosome.

The term "plasmid copy number" refers to the number of plasmids in a genome of a specific organism. Under the control of a plasmid replicon, the plasmid copy number may fluctuate in a narrow range with changes of bacterial culture conditions. When growth conditions are constant, the speed of plasmid proliferation is exactly the same as the speed of host cell proliferation, and the copy number remains unchanged.

The term "single-copy plasmid" means that there is only one specific plasmid in a biological genome.

The term "competent" refers to a special physiological state in which a cell can take up DNA molecules from a surrounding environment and the DNA molecules are not easily broken down by restriction endonucleases in the cell.

The term "glucose" refers to a polyhydroxy aldehyde with the molecular formula C₆H₁₂O₆, which is important in the field of biology and is an energy source for living cells and an intermediate product of metabolism.

The term "arabinose" is also referred to as L(+)-arabinose, pectinose, or the like, with the molecular formula C₅H₁₀O₅, which is a levorotatory monosaccharide.

The term "tryptone" is also referred to as casein tryptone and pancreatic digest of casein, which is a high-quality peptone and is light yellow powder formed through concentration and drying. The tryptone is rich in nitrogen sources, amino acids and the like, can be used to prepare various microbial culture media for culture, isolation, proliferation, and identification of bacteria, and can be used to prepare bacterial biochemical characteristics test culture media such as a sterile test culture medium and an anaerobic bacteria culture medium.

The term "yeast extract" is also referred to as yeast flavor, which is abbreviated as YE according to international usage. Main components of the yeast extract are polypeptides, amino acids, 5'-ribonucleotide, B vitamins, and trace elements. The yeast extract is the most ideal raw material for biological culture media and the main raw material in fermentation industries. The yeast extract has the efficacy 8 times that of yeast, which can greatly increase the production rate of bacterial strains and the yield of fermentation products.

### BRIEF DESCRIPTION OF THE DRAWINGS

FIG. 1 shows a result of plasmid extraction in Example 1, where a horizontal coordinate is a sampling time, and a longitudinal coordinate is a concentration of extracted plasmids;
FIG. 2 shows a result of plasmid extraction in Example 2, where a horizontal coordinate is a sampling time, and a longitudinal coordinate is a concentration of extracted plasmids;
FIG. 3 shows a result of plasmid extraction in Example 3, where a horizontal coordinate is a sampling time, and a longitudinal coordinate is a concentration of extracted plasmids;
FIG. 4 shows a result of plasmid extraction in Example 4, where a horizontal coordinate is a sampling time, and a longitudinal coordinate is a concentration of extracted plasmids;
FIG. 5 shows a result of plasmid extraction in Example 5, where a horizontal coordinate is a sampling time, and a longitudinal coordinate is a concentration of extracted plasmids;
FIG. 6 shows a result of plasmid extraction in Example 6, where a horizontal coordinate is a sampling time, and a longitudinal coordinate is a concentration of extracted plasmids;
FIG. 7 shows a result of plasmid extraction in Comparative Example 1, where a horizontal coordinate is a sampling time, and a longitudinal coordinate is a concentration of extracted plasmids; and
FIG. 8 is a diagram of a pCCIBAC vector.

### DETAILED DESCRIPTION

The present invention is further described below with reference to the specific implementations.

Unless otherwise specified, the experimental methods used in the following examples are all conventional methods.

Unless otherwise specified, the materials and reagents used in the following examples can be commercially available.

### Example 1

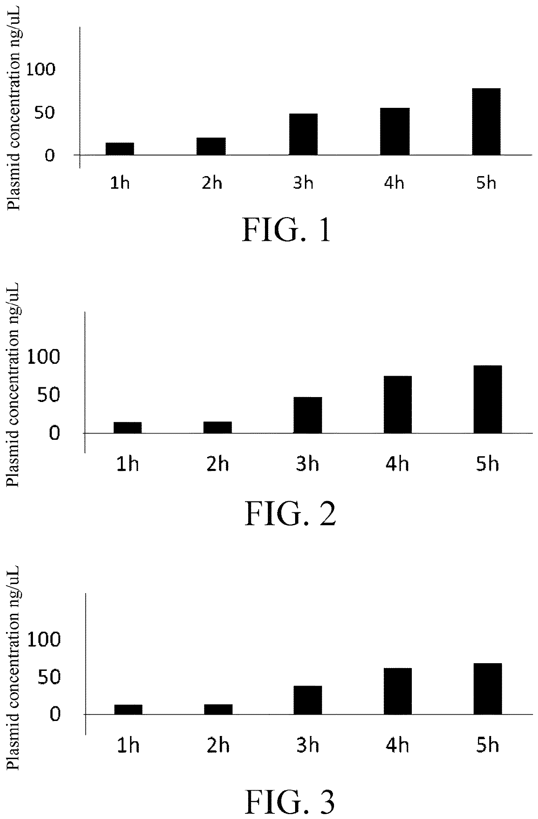

The pCCIBAC plasmid (shown as SEQ ID NO: 1) included a replication origin oriV. The pCCIBAC plasmid was transformed into competent EPI300 *Escherichia coli* by chemical transformation. The transformed *Escherichia coli* was cultured in the LB culture medium (in which components were 10 g/L of tryptone, 5 g/L of yeast extract, and 10 g/L of sodium chloride) overnight, and then was inoculated at an inoculation rate of 5% into a culture medium in which components were 10 g/L of tryptone, 5 g/L of yeast extract, 10 g/L of sodium chloride, 2 g/L of glucose, and 0.75 g/L of arabinose. The bacteria were cultured at 220 r/min and 25°C for 5 h. After the inoculation, 6 OD600 bacteria were taken every 1 h for plasmid extraction. The plasmid extraction was performed by using the standard procedure of the plasmid extraction kit AxyGEN Miniprep Kit (Lot#17718KA1). The result of the plasmid extraction was shown in FIG. 1.

### Example 2

The pCCIBAC plasmid included a replication origin oriV. The plasmid was transformed into competent EPI400 *Escherichia coli* by chemical transformation. The transformed *Escherichia coli* was cultured in the LB culture medium (in which components were 10 g/L of tryptone, 5 g/L of yeast extract, and 10 g/L of sodium chloride) overnight, and then was inoculated at an inoculation rate of 5% into a culture medium in which components were 5 g/L of tryptone, 10 g/L of yeast extract, 5 g/L of sodium chloride, 1 g/L of glucose, and 0.6 g/L of arabinose. The bacteria were cultured at 220 r/min and 30°C for 5 h. After the inoculation, 6 OD600 bacteria were taken every 1 h for plasmid extraction. The plasmid extraction was performed by using the standard procedure of the plasmid extraction kit AxyGEN Miniprep Kit (Lot#17718KA1). The result of the plasmid extraction was shown in FIG. 2.

### Example 3

The pCCIBAC plasmid included a replication origin oriV. The pCCIBAC plasmid was transformed into competent EPI300 *Escherichia coli* by chemical transformation. The transformed *Escherichia coli* was cultured in the LB culture medium (in which components were 10 g/L of tryptone, 5 g/L of yeast extract, and 10 g/L of sodium chloride) overnight, and then was inoculated at an inoculation rate of 5% into a culture medium in which components were 10 g/L of tryptone, 10 g/L of yeast extract, 10 g/L of sodium chloride, 0.5 g/L of glucose, and 0.3 g/L of arabinose. The bacteria were cultured at 220 r/min and 37°C for 5 h. After the inoculation, 6 OD600 bacteria were taken every 1 h for plasmid extraction. The plasmid extraction was performed by using the standard procedure of the plasmid extraction kit AxyGEN Miniprep Kit (Lot#17718KA1). The result of the plasmid extraction was shown in FIG. 3.

### Example 4

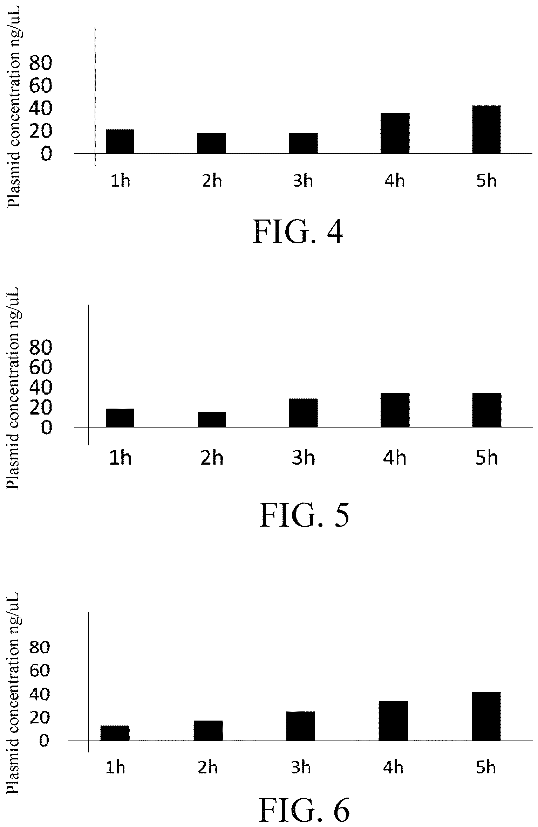

The pCCIBAC plasmid included a replication origin oriV. The plasmid was transformed into competent EPI400 *Escherichia coli* by chemical transformation. The transformed *Escherichia coli* was cultured in the LB culture medium (in which components were 10 g/L of tryptone, 5 g/L of yeast extract, and 10 g/L of sodium chloride) overnight, and then was inoculated at an inoculation rate of 5% into a culture medium in which components were 10 g/L of tryptone, 10 g/L of yeast extract, 5 g/L of sodium chloride, 2 g/L of glucose, and 1 g/L of arabinose. The bacteria were cultured at 220 r/min and 30°C for 5 h. After the inoculation, 6 OD600 bacteria were taken every 1 h for plasmid extraction. The plasmid extraction was performed by using the standard procedure of the plasmid extraction kit AxyGEN Miniprep Kit (Lot#17718KA1). The result of the plasmid extraction was shown in FIG. 4.

### Example 5

The pCCIBAC plasmid included a replication origin oriV. The plasmid was transformed into competent EPI400 *Escherichia coli* by chemical transformation. The transformed *Escherichia coli* was cultured in the LB culture medium (in which components were 10 g/L of tryptone, 5 g/L of yeast extract, and 10 g/L of sodium chloride) overnight, and then was inoculated at an inoculation rate of 5% into a culture medium in which components were 10 g/L of tryptone, 10 g/L of yeast extract, 5 g/L of sodium chloride, 10 g/L of glucose, and 5 g/L of arabinose. The bacteria were cultured at 220 r/min and 30°C for 5 h. After the inoculation, 6 OD600 bacteria were taken every 1 h for plasmid extraction. The plasmid extraction was performed by using the standard procedure of the plasmid extraction kit AxyGEN Miniprep Kit (Lot#17718KA1). The result of the plasmid extraction was shown in FIG. 5.

### Example 6

The pCCIBAC plasmid included a replication origin oriV. The plasmid was transformed into competent EPI400 *Escherichia coli* by chemical transformation. The transformed *Escherichia coli* was cultured in the LB culture medium (in which components were 10 g/L of tryptone, 5 g/L of yeast extract, and 10 g/L of sodium chloride) overnight, and then was inoculated at an inoculation rate of 5% into a culture medium in which components were 10 g/L of tryptone, 10 g/L of yeast extract, 5 g/L of sodium chloride, 10 g/L of glucose, and 2 g/L of arabinose. The bacteria were cultured at 220 r/min and 30°C for 5 h. After the inoculation, 6 OD600 bacteria were taken every 1 h for plasmid extraction. The plasmid extraction was performed by using the standard procedure of the plasmid extraction kit AxyGEN Miniprep Kit (Lot#17718KA1). The result of the plasmid extraction was shown in FIG. 6.

### Comparative Example 1 (without glucose and arabinose)

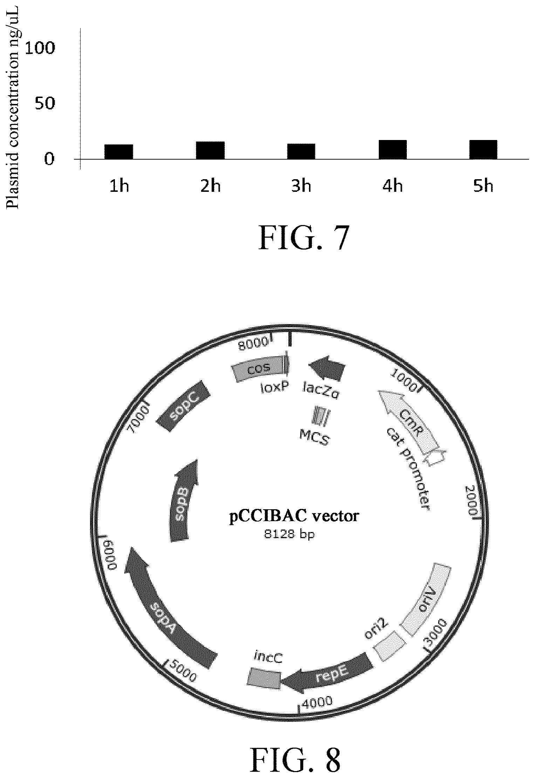

The pCCIBAC plasmid included a replication origin oriV. The pCCIBAC plasmid was transformed into competent EPI300 *Escherichia coli* by chemical transformation. The transformed *Escherichia coli* was cultured in the LB culture medium (in which components were 10 g/L of tryptone, 5 g/L of yeast extract, and 10 g/L of sodium chloride) overnight, and then was inoculated at an inoculation rate of 5% into a culture medium in which components were 10 g/L of tryptone, 10 g/L of yeast extract, and 10 g/L of sodium chloride. The bacteria were cultured at 220 r/min and 30°C. After the inoculation, 6 OD600 bacteria were taken every 1 h for plasmid extraction. The plasmid extraction was performed by using the standard procedure of the plasmid extraction kit AxyGEN Miniprep Kit (Lot#17718KA1). The result of the plasmid extraction was shown in FIG. 7.

### Comparative Example 2 (conventional method for inducing a plasmid copy number)

The pCCIBAC plasmid included a replication origin oriV. The pCCIBAC plasmid was transformed into competent EPI300 *Escherichia coli* by chemical transformation. The transformed *Escherichia coli* was cultured in the LB culture medium (in which components were 10 g/L of tryptone, 5 g/L of yeast extract, and 10 g/L of sodium chloride) overnight, and then was inoculated at an inoculation rate of 5% into a culture medium in which components were 10 g/L of tryptone, 10 g/L of yeast extract, and 10 g/L of sodium chloride. The bacteria were cultured at 220 r/min and 30°C for 12 h, and then, were used as a seed solution. Re-inoculation was performed with the seed solution, and OD600 after the re-inoculation was 0.2. Then, the bacteria were cultured at 30°C for 1 h, and arabinose was added to make the concentration be 0.2 g/L. The culture was continued for 5 h, 6 OD600 bacteria were taken every 1 h for plasmid extraction. The plasmid extraction was performed by using the standard procedure of the plasmid extraction kit AxyGEN Miniprep Kit (Lot#17718KA1). The result of the plasmid extraction was shown in FIG. 8.

After the plasmid extraction, plasmid concentrations were measured by using Nano Drop oneC (Thermo Fisher Scientific). Results were shown in Table 1.

**Table 1 Plasmid extraction concentration (ng/µL)**

| Time | 1 h | 2 h | 3 h | 4 h | 5 h |
|---|---|---|---|---|---|
| Comparative Example 1 | 12.5 | 15.4 | 13.2 | 16.4 | 16.4 |
| Example 1 | 14.5 | 20.2 | 48.6 | 55.4 | 78.5 |
| Example 2 | 14.3 | 15.4 | 46.4 | 74.5 | 88.5 |
| Example 3 | 12.8 | 13.3 | 37.8 | 62.3 | 68.4 |
| Example 4 | 21.2 | 18.3 | 18.4 | 35.6 | 42.2 |
| Example 5 | 18.4 | 15.3 | 28.4 | 34.1 | 34.4 |
| Example 6 | 13.1 | 17.4 | 25.2 | 33.8 | 41.5 |
| Comparative Example 2 | 12.5 | 24.8 | 27.8 | 38.3 | 58.4 |

The comparison of the plasmid extraction concentrations between Examples 1 to 6 and Comparative Example 1 was shown in Table 2. After glucose and arabinose were added into the culture medium, the concentration of the extracted plasmids increased rapidly. After culture in the culture medium for 5 h, the plasmid extraction concentrations increased by from 110 to 440%. Especially, when the concentration of glucose was from 0.5 to 2 g/L and the concentration of arabinose was from 0.3 to 0.75 g/L, after culture in the culture medium for 5 h, the plasmid extraction concentrations increased by from 317 to 440%. It indicates that the culture medium provided by the present invention is suitable for inducing an increase in a plasmid copy number and greatly improving the plasmid yield.

**Table 2 Percentages of increases of plasmid extraction concentrations of Examples 1 to 6 compared with Comparative Example 1**

| Time | 1 h | 2 h | 3 h | 4 h | 5 h |
|---|---|---|---|---|---|
| Example 1 | 16% | 31% | 268% | 238% | 379% |
| Example 2 | 14% | 0% | 252% | 354% | 440% |
| Example 3 | 2% | -14% | 186% | 280% | 317% |
| Example 4 | 70% | 19% | 39% | 117% | 157% |
| Example 5 | 47% | -1% | 115% | 108% | 110% |
| Example 6 | 5% | 13% | 91% | 106% | 153% |

Compared with Comparative Example 2 (the conventional method for inducing a plasmid copy number), Examples 1 to 6 had simple operations and greatly improved plasmid extraction concentrations. As shown in Table 3, when the concentration of glucose was from 0.5 to 2 g/L and the concentration of arabinose was from 0.3 to 0.75 g/L, after extraction for 3 h, the plasmid extraction concentrations increased by from 36 to 75% compared to the conventional method, and after extraction for 4 h, the plasmid extraction concentrations increased by from 45 to 95% compared to the conventional method. Especially, when the concentration of glucose was 1 g/L and the concentration of arabinose was 0.6 g/L, after extraction for 4 h, the plasmid extraction concentrations increased by 95% compared to the conventional method.

**Table 3 Percentages of increases of plasmid extraction concentrations of Examples 1 to 6 compared with Comparative Example 2**

| Time | 1 h | 2 h | 3 h | 4 h | 5 h |
|---|---|---|---|---|---|
| Example 1 | 16% | -19% | 75% | 45% | 34% |
| Example 2 | 14% | -38% | 67% | 95% | 52% |
| Example 3 | 2% | -46% | 36% | 63% | 17% |
| Example 4 | 70% | -26% | -34% | -7% | -28% |
| Example 5 | 47% | -38% | 2% | -11% | -41% |
| Example 6 | 5% | -30% | -9% | -12% | -29% |

SEQ ID NO: 1 pCCIBAC vector sequence

gcggccgcaaggggttcgcgtcagcgggtgttggcgggtgtcggggctggcttaactatgcggcatcagagc
agattgtactgagagtgcaccatatgcggtgtgaaataccacacagatgcgtaaggagaaaataccgcatcag
gcgccattcgccattcagctgcgcaactgttgggaagggcgatcggtgcgggcctcttcgctattacgccagct
ggcgaaagggggatgtgctgcaaggcgattaagttgggtaacgccagggttttcccagtcacgacgttgtaaa
acgacggccagtgaattgtaatacgactcactatagggcgaattcgagctcggtacccggggatcctctagagt
cgacctgcaggcatgcaagcttgagtattctatagtctcacctaaatagcttggcgtaatcatggtcatagctgttt
cctgtgtgaaattgttatccgctcacaattccacacaacatacgagccggaagcataaagtgtaaagcctggggt
gcctaatgagtgagctaactcacattaattgcgttgcgctcactgcccgctttccagtcgggaaacctgtcgtgcc
agctgcattaatgaatcggccaacgcgaaccccttgcggccgcccgggccgtcgaccaattctcatgtttgaca
gcttatcatcgaatttctgccattcatccgcttattatcacttattcaggcgtagcaaccaggcgtttaagggcacca
ataactgccttaaaaaaattacgccccgccctgccactcatcgcagtactgttgtaattcattaagcattctgccga
catggaagccatcacaaacggcatgatgaacctgaatcgccagcggcatcagcaccttgtcgccttgcgtataa
tatttgcccatggtgaaaacgggggcgaagaagttgtccatattggccacgtttaaatcaaaactggtgaaactc
acccagggattggctgagacgaaaaacatattctcaataaaccctttagggaaataggccaggttttcaccgtaa
cacgccacatcttgcgaatatatgtgtagaaactgccggaaatcgtcgtggtattcactccagagcgatgaaaac
gtttcagtttgctcatggaaaacggtgtaacaagggtgaacactatcccatatcaccagctcaccgtctttcattgc
catacgaaattccggatgagcattcatcaggcgggcaagaatgtgaataaaggccggataaaacttgtgcttatt
tttctttacggtctttaaaaaggccgtaatatccagctgaacggtctggttataggtacattgagcaactgactgaa
atgcctcaaaatgttctttacgatgccattgggatatatcaacggtggtatatccagtgatttttttctccattttagctt
ccttagctcctgaaaatctcgataactcaaaaaatacgcccggtagtgatcttatttcattatggtgaaagttggaa
cctcttacgtgccgatcaacgtctcattttcgccaaaagttggcccagggcttcccggtatcaacagggacacca
ggatttatttattctgcgaagtgatcttccgtcacaggtatttattcgcgataagctcatggagcggcgtaaccgtc
gcacaggaaggacagagaaagcgcggatctgggaagtgacggacagaacggtcaggacctggattgggg
aggcggttgccgccgctgctgctgacggtgtgacgttctctgttccggtcacaccacatacgttccgccattcct
atgcgatgcacatgctgtatgccggtataccgctgaaagttctgcaaagcctgatgggacataagtccatcagtt

caacggaagtctacacgaaggtttttgcgctggatgtggctgcccggcaccgggtgcagtttgcgatgccgga
gtctgatgcggttgcgatgctgaaacaattatcctgagaataaatgccttggcctttatatggaaatgtggaactga
gtggatatgctgtttttgtctgttaaacagagaagctggctgttatccactgagaagcgaacgaaacagtcggga
aaatctcccattatcgtagagatccgcattattaatctcaggagcctgtgtagcgtttataggaagtagtgttctgtc
atgatgcctgcaagcggtaacgaaaacgatttgaatatgccttcaggaacaatagaaatcttcgtgcggtgttac
gttgaagtggagcggattatgtcagcaatggacagaacaacctaatgaacacagaaccatgatgtggtctgtcc
ttttacagccagtagtgctcgccgcagtcgagcgacagggcgaagccctcgagctggttgccctcgccgctgg
gctggcggccgtctatggccctgcaaacgcgccagaaacgccgtcgaagccgtgtgcgagacaccgcggc
cggccgccggcgttgtggatacctcgcggaaaacttggccctcactgacagatgaggggcggacgttgacac
ttgaggggccgactcacccggcgcggcgttgacagatgaggggcaggctcgatttcggccggcgacgtgga
gctggccagcctcgcaaatcggcgaaaacgcctgattttacgcgagtttcccacagatgatgtggacaagcctg
gggataagtgccctgcggtattgacacttgaggggcgcgactactgacagatgaggggcgcgatccttgaca
cttgaggggcagagtgctgacagatgaggggcgcacctattgacatttgaggggctgtccacaggcagaaaa
tccagcatttgcaagggtttccgcccgtttttcggccaccgctaacctgtcttttaacctgcttttaaaccaatatttat
aaaccttgtttttaaccagggctgcgccctgtgcgcgtgaccgcgcacgccgaaggggggtgcccccccttct
cgaaccctcccggtcgagtgagcgaggaagcaccagggaacagcacttatatattctgcttacacacgatgcc
tgaaaaaacttcccttggggttatccacttatccacggggatatttttataattattttttttatagtttttagatcttcttttt
tagagcgccttgtaggcctttatccatgctggttctagagaaggtgttgtgacaaattgccctttcagtgtgacaaa

tcaccctcaaatgacagtcctgtctgtgacaaattgcccttaaccctgtgacaaattgccctcagaagaagctgttt
tttcacaaagttatccctgcttattgactctttttatttagtgtgacaatctaaaaacttgtcacacttcacatggatctg
tcatggcggaaacagcggttatcaatcacaagaaacgtaaaaatagcccgcgaatcgtccagtcaaacgacct
cactgaggcggcatatagtctctcccgggatcaaaaacgtatgctgtatctgttcgttgaccagatcagaaaatct
gatggcaccctacaggaacatgacggtatctgcgagatccatgttgctaaatatgctgaaatattcggattgacct
ctgcggaagccagtaaggatatacggcaggcattgaagagtttcgcggggaaggaagtggtttttatcgccct
gaagaggatgccggcgatgaaaaaggctatgaatcttttccttggtttatcaaacgtgcgcacagtccatccaga
gggctttacagtgtacatatcaacccatatctcattcccttctttatcgggttacagaaccggtttacgcagtttcgg
cttagtgaaacaaaagaaatcaccaatccgtatgccatgcgtttatacgaatccctgtgtcagtatcgtaagccgg
atggctcaggcatcgtctctctgaaaatcgactggatcatagagcgttaccagctgcctcaaagttaccagcgta
tgcctgacttccgccgccgcttcctgcaggtctgtgttaatgagatcaacagcagaactccaatgcgcctctcata
cattgagaaaaagaaaggccgccagacgactcatatcgtattttccttccgcgatatcacttccatgacgacagg
atagtctgagggttatctgtcacagatttgagggtggttcgtcacatttgttctgacctactgagggtaatttgtcac
agttttgctgtttccttcagcctgcatggatttctcatactttttgaactgtaattttaaggaagccaaatttgagggc
agtttgtcacagttgatttccttctctttcccttcgtcatgtgacctgatatcgggggttagttcgtcatcattgatgag
ggttgattatcacagtttattactctgaattggctatccgcgtgtgtacctctacctggagtttttcccacggtggata
tttcttcttgcgctgagcgtaagagctatctgacagaacagttcttctttgcttcctcgccagttcgctcgctatgctc
ggttacacggctgcggcgagcgctagtgataataagtgactgaggtatgtgctcttcttatctccttttgtagtgttg
ctcttattttaaacaactttgcggtttttttgatgactttgcgattttgttgttgctttgcagtaaattgcaagatttaataa
aaaacgcaaagcaatgattaaaggatgttcagaatgaaactcatggaaacacttaaccagtgcataaacgctgg
tcatgaaatgacgaaggctatcgccattgcacagtttaatgatgacagcccggaagcgaggaaaataacccgg
cgctggagaataggtgaagcagcggatttagttggggtttcttctcaggctatcagagatgccgagaaagcagg
gcgactaccgcacccggatatggaaattcgaggacgggttgagcaacgtgttggttatacaattgaacaaatta
atcatatgcgtgatgtgtttggtacgcgattgcgacgtgctgaagacgtatttccaccggtgatcggggttgctgc
ccataaaggtggcgtttacaaaacctcagtttctgttcatcttgctcaggatctggctctgaagggggctacgtgtttt

gctcgtggaaggtaacgacccccagggaacagcctcaatgtatcacggatgggtaccagatcttcatattcatg
cagaagacactctcctgcctttctatcttggggaaaaggacgatgtcacttatgcaataaagcccacttgctggcc
ggggcttgacattattccttcctgtctggctctgcaccgtattgaaactgagttaatgggcaaatttgatgaaggta
aactgcccaccgatccacacctgatgctccgactggccattgaaactgttgctcatgactatgatgtcatagttatt
gacagcgcgcctaacctgggtatcggcacgattaatgtcgtatgtgctgctgatgtgctgattgttcccacgcctg
ctgagttgtttgactacacctccgcactgcagtttttcgatatgcttcgtgatctgctcaagaacgttgatcttaaag
ggttcgagcctgatgtacgtattttgcttaccaaatacagcaatagcaatggctctcagtccccgtggatggagg
agcaaattcgggatgcctggggaagcatggttctaaaaaatgttgtacgtgaaacggatgaagttggtaaaggt
cagatccggatgagaactgtttttgaacaggccattgatcaacgctcttcaactggtgcctggagaaatgctcttt
ctatttgggaacctgtctgcaatgaaattttcgatcgtctgattaaaccacgctgggagattagataatgaagcgtg
cgcctgttattccaaaacatacgctcaatactcaaccggttgaagatacttcgttatcgacaccagctgccccgat
ggtggattcgttaattgcgcgcgtaggagtaatggctcgcggtaatgccattactttgcctgtatgtggtcgggat
gtgaagtttactcttgaagtgctccggggtgatagtgttgagaagacctctcgggtatggtcaggtaatgaacgt
gaccaggagctgcttactgaggacgcactggatgatctcatcccttcttttctactgactggtcaacagacaccg
gcgttcggtcgaagagtatctggtgtcatagaaattgccgatgggagtcgccgtcgtaaagctgctgcacttacc
gaaagtgattatcgtgttctggttggcgagctggatgatgagcagatggctgcattatccagattgggtaacgatt
atcgcccaacaagtgcttatgaacgtggtcagcgttatgcaagccgattgcagaatgaatttgctggaaatatttc
tgcgctggctgatgcggaaaatatttcacgtaagattattacccgctgtatcaacaccgccaaattgcctaaatca
gttgttgctctttttctcaccccggtgaactatctgcccggtcaggtgatgcacttcaaaaagcctttacagataaa
gaggaattacttaagcagcaggcatctaaccttcatgagcagaaaaagctggggtgatatttgaagctgaaga
agttatcactcttttaacttctgtgcttaaaacgtcatctgcatcaagaactagtttaagctcacgacatcagtttgctc
ctggagcgacagtattgtataagggcgataaaatggtgcttaacctggacaggtctcgtgttccaactgagtgtat
agagaaaattgaggccattcttaaggaacttgaaaagccagcaccctgatgcgaccacgttttagtctacgtttat
ctgtctttacttaatgtcctttgttacaggccagaaagcataactggcctgaatattctctctgggcccactgttcca
cttgtatcgtcggtctgataatcagactgggaccacggtcccactcgtatcgtcggtctgattattagtctgggacc
acggtcccactcgtatcgtcggtctgattattagtctgggaccacggtcccactcgtatcgtcggtctgataatca

gactgggaccacggtcccactcgtatcgtcggtctgattattagtctgggaccatggtcccactcgtatcgtcggt
ctgattattagtctgggaccacggtcccactcgtatcgtcggtctgattattagtctggaaccacggtcccactcgt
atcgtcggtctgattattagtctgggaccacggtcccactcgtatcgtcggtctgattattagtctgggaccacgat
cccactcgtgttgtcggtctgattatcggtctgggaccacggtcccacttgtattgtcgatcagactatcagcgtga
gactacgattccatcaatgcctgtcaagggcaagtattgacatgtcgtcgtaacctgtagaacggagtaacctcg
gtgtgcggttgtatgcctgctgtggattgctgctgtgtcctgcttatccacaacattttgcgcacggttatgtggaca
aaatacctggttacccaggccgtgccggcacgttaaccgggctgcatccgatgcaagtgtgtcgctgtcgacg
agctcgcgagctcggacatgaggttgccccgtattcagtgtcgctgatttgtattgtctgaagttgtttttacgttaa
gttgatgcagatcaattaatacgatacctgcgtcataattgattatttgacgtggtttgatggcctccacgcacgttg
tgatatgtagatgataatcattatcactttacgggtcctttccggtgatccgacaggttacggggcggcgacctcg
cgggttttcgctatttatgaaaattttccggtttaaggcgtttccgttcttcttcgtcataacttaatgtttttatttaaaat
accctctgaaaagaaaggaaacgacaggtgctgaaagcgagctttttggcctctgtcgtttcctttctctgtttttgt
ccgtggaatgaacaatggaagtccgagctcatcgctaataacttcgtatagcatacattatacgaagttatattcg
at

The culture medium for inducing an increase in a plasmid copy number and use thereof provided in the present invention are described in detail above. The principle and implementations of the present invention are described herein through specific examples. The description about the foregoing examples is merely provided to help understand the method and core ideas of the present invention. It should be noted that a person skilled in the art may further make several improvements and modifications to the present invention without departing from the principle of the present invention. These improvements and modifications also fall within the protection scope of the claims of the present invention.

## Claims

1. A culture medium for inducing an increase in a plasmid copy number, comprising tryptone, a yeast extract, glucose, sodium chloride, and arabinose.

2. The culture medium for inducing an increase in a plasmid copy number according to claim 1, wherein the glucose has a concentration of from 0.1 to 10 g/L, preferably from 1 to 5 g/L, and more preferably from 0.5 to 2 g/L.

3. The culture medium for inducing an increase in a plasmid copy number according to claim 1, wherein the arabinose has a concentration of from 0.1 to 10 g/L, preferably from 1 to 5 g/L, and more preferably from 0.3 to 0.75 g/L.

4. The culture medium for inducing an increase in a plasmid copy number according to claim 1, wherein the tryptone has a concentration of from 1 to 15 g/L, and preferably from 5 to 10 g/L.

5. The culture medium for inducing an increase in a plasmid copy number according to claim 1, wherein the yeast extract has a concentration of from 1 to 15 g/L, and preferably from 5 to 10 g/L.

6. The culture medium for inducing an increase in a plasmid copy number according to claim 1, wherein the sodium chloride has a concentration of from 1 to 15 g/L, and preferably from 5 to 10 g/L.

7. The culture medium for inducing an increase in a plasmid copy number according to any one of claims 1 to 6, further comprising one or more of magnesium chloride, potassium chloride, ferric chloride, or calcium chloride.

8. The culture medium for inducing an increase in a plasmid copy number according to any one of claims 1 to 7, wherein the plasmid is a plasmid comprising a replication origin onV.

9. The culture medium for inducing an increase in a plasmid copy number according to claim 8, wherein the plasmid is a single-copy plasmid comprising a replication origin oriV, preferably a pCCIBAC plasmid.

10. A method for inducing an increase in a plasmid copy number, comprising the following steps:
Step (1): transforming *Escherichia coli* with a plasmid; and
Step (2): inoculating the *Escherichia coli* obtained in Step (1) into the culture medium according to any one of claims 1 to 9 for culturing under conditions suitable for culture.

11. The method for inducing an increase in a plasmid copy number according to claim 10, wherein in Step (1), the plasmid is transformed into competent *Escherichia coli.*

12. The method for inducing an increase in a plasmid copy number according to claim 10, wherein in Step (1), the *Escherichia coli* is EPI300 or EPI400 *Escherichia coli.*

13. The method for inducing an increase in a plasmid copy number according to claim 10, wherein in Step (2), a culture temperature is from 25 to 37 °C, and a culture time is from 4 to 6 h.

14. Use of the culture medium according to any one of claims 1 to 9 in inducing an increase in a plasmid copy number.


<110>

<120>

<130>  OP191220

<150>  201811610822.6
<151>  2018-12-27

<160>  1

<170>  SIPOSequenceListing 1.0

<210>  1
<211>  8128
<212>  DNA
<213>  (Artificial Sequence)

<400>  1
gcggccgcaa ggggttcgcg tcagcgggtg ttggcgggtg tcggggctgg cttaactatg      60
cggcatcaga gcagattgta ctgagagtgc accatatgcg gtgtgaaata ccacacagat     120
gcgtaaggag aaaataccgc atcaggcgcc attcgccatt cagctgcgca actgttggga     180
agggcgatcg gtgcgggcct cttcgctatt acgccagctg gcgaaagggg gatgtgctgc     240
aaggcgatta agttgggtaa cgccagggtt ttcccagtca cgacgttgta aaacgacggc     300
cagtgaattg taatacgact cactataggg cgaattcgag ctcggtaccc ggggatcctc     360
tagagtcgac ctgcaggcat gcaagcttga gtattctata gtctcaccta aatagcttgg     420
cgtaatcatg gtcatagctg tttcctgtgt gaaattgtta tccgctcaca attccacaca     480
acatacgagc cggaagcata aagtgtaaag cctggggtgc ctaatgagtg agctaactca     540
cattaattgc gttgcgctca ctgcccgctt tccagtcggg aaacctgtcg tgccagctgc     600
attaatgaat cggccaacgc gaaccccttg cggccgcccg ggccgtcgac caattctcat     660
gtttgacagc ttatcatcga atttctgcca ttcatccgct tattatcact tattcaggcg     720
tagcaaccag gcgtttaagg gcaccaataa ctgccttaaa aaaattacgc cccgccctgc     780
cactcatcgc agtactgttg taattcatta agcattctgc cgacatggaa gccatcacaa     840
acggcatgat gaacctgaat cgccagcggc atcagcacct tgtcgccttg cgtataatat     900
ttgcccatgg tgaaaacggg ggcgaagaag ttgtccatat tggccacgtt taaatcaaaa     960
ctggtgaaac tcacccaggg attggctgag acgaaaaaca tattctcaat aaacccttta    1020
gggaaatagg ccaggttttc accgtaacac gccacatctt gcgaatatat gtgtagaaac    1080
tgccggaaat cgtcgtggta ttcactccag agcgatgaaa acgtttcagt ttgctcatgg    1140
aaaacggtgt aacaagggtg aacactatcc catatcacca gctcaccgtc tttcattgcc    1200
atacgaaatt ccggatgagc attcatcagg cgggcaagaa tgtgaataaa ggccggataa    1260
aacttgtgct tatttttctt tacggtcttt aaaaaggccg taatatccag ctgaacggtc    1320
tggttatagg tacattgagc aactgactga aatgcctcaa aatgttcttt acgatgccat    1380
tgggatatat caacggtggt atatccagtg atttttttct ccattttagc ttccttagct    1440
cctgaaaatc tcgataactc aaaaaatacg cccggtagtg atcttatttc attatggtga    1500
aagttggaac ctcttacgtg ccgatcaacg tctcattttc gccaaaagtt ggcccagggc    1560
ttcccggtat caacagggac accaggattt atttattctg cgaagtgatc ttccgtcaca    1620
ggtatttatt cgcgataagc tcatggagcg gcgtaaccgt cgcacaggaa ggacagagaa    1680
agcgcggatc tgggaagtga cggacagaac ggtcaggacc tggattgggg aggcggttgc    1740
cgccgctgct gctgacggtg tgacgttctc tgttccggtc acaccacata cgttccgcca    1800
ttcctatgcg atgcacatgc tgtatgccgg tataccgctg aaagttctgc aaagcctgat    1860
gggacataag tccatcagtt caacggaagt ctacacgaag gtttttgcgc tggatgtggc    1920
tgcccggcac cgggtgcagt ttgcgatgcc ggagtctgat gcggttgcga tgctgaaaca    1980
attatcctga gaataaatgc cttggccttt atatggaaat gtggaactga gtggatatgc    2040
tgtttttgtc tgttaaacag agaagctggc tgttatccac tgagaagcga acgaaacagt    2100
cgggaaaatc tcccattatc gtagagatcc gcattattaa tctcaggagc ctgtgtagcg    2160
tttataggaa gtagtgttct gtcatgatgc ctgcaagcgg taacgaaaac gatttgaata    2220
tgccttcagg aacaatagaa atcttcgtgc ggtgttacgt tgaagtggag cggattatgt    2280
cagcaatgga cagaacaacc taatgaacac agaaccatga tgtggtctgt ccttttacag    2340
ccagtagtgc tcgccgcagt cgagcgacag ggcgaagccc tcgagctggt tgccctcgcc    2400
gctgggctgg cggccgtcta tggccctgca aacgcgccag aaacgccgtc gaagccgtgt    2460
gcgagacacc gcggccggcc gccggcgttg tggatacctc gcggaaaact tggccctcac    2520

tgacagatga ggggcggacg ttgacacttg aggggccgac tcacccggcg cggcgttgac 2580
agatgagggg caggctcgat ttcggccggc gacgtggagc tggccagcct cgcaaatcgg 2640
cgaaaacgcc tgattttacg cgagtttccc acagatgatg tggacaagcc tggggataag 2700
tgccctgcgg tattgacact tgaggggcgc gactactgac agatgagggg cgcgatcctt 2760
gacacttgag gggcagagtg ctgacagatg aggggcgcac ctattgacat ttgaggggct 2820
gtccacaggc agaaaatcca gcatttgcaa gggtttccgc ccgtttttcg gccaccgcta 2880
acctgtcttt taacctgctt ttaaaccaat atttataaac cttgttttta accagggctg 2940
cgccctgtgc gcgtgaccgc gcacgccgaa ggggggtgcc cccccttctc gaaccctccc 3000
ggtcgagtga gcgaggaagc accagggaac agcacttata tattctgctt acacacgatg 3060
cctgaaaaaa cttcccttgg ggttatccac ttatccacgg ggatattttt ataattattt 3120
tttttatagt ttttagatct tcttttttag agcgccttgt aggcctttat ccatgctggt 3180
tctagagaag gtgttgtgac aaattgccct ttcagtgtga caaatcaccc tcaaatgaca 3240
gtcctgtctg tgacaaattg cccttaaccc tgtgacaaat tgccctcaga agaagctgtt 3300
ttttcacaaa gttatccctg cttattgact cttttttatt tagtgtgaca atctaaaaac 3360
ttgtcacact tcacatggat ctgtcatggc ggaaacagcg gttatcaatc acaagaaacg 3420
taaaaatagc ccgcgaatcg tccagtcaaa cgacctcact gaggcggcat atagtctctc 3480
ccgggatcaa aaacgtatgc tgtatctgtt cgttgaccag atcagaaaat ctgatggcac 3540
cctacaggaa catgacggta tctgcgagat ccatgttgct aaatatgctg aaatattcgg 3600
attgacctct gcggaagcca gtaaggatat acggcaggca ttgaagagtt tcgcggggaa 3660
ggaagtggtt ttttatcgcc ctgaagagga tgccggcgat gaaaaaggct atgaatcttt 3720
tccttggttt atcaaacgtg cgcacagtcc atccagaggg ctttacagtg tacatatcaa 3780
cccatatctc attcccttct ttatcgggtt acagaaccgg tttacgcagt ttcggcttag 3840
tgaaacaaaa gaaatcacca atccgtatgc catgcgttta tacgaatccc tgtgtcagta 3900
tcgtaagccg gatggctcag gcatcgtctc tctgaaaatc gactggatca tagagcgtta 3960
ccagctgcct caaagttacc agcgtatgcc tgacttccgc cgccgcttcc tgcaggtctg 4020
tgttaatgag atcaacagca gaactccaat gcgcctctca tacattgaga aaaagaaagg 4080
ccgccagacg actcatatcg tattttcctt ccgcgatatc acttccatga cgacaggata 4140
gtctgagggt tatctgtcac agatttgagg gtggttcgtc acatttgttc tgacctactg 4200
agggtaattt gtcacagttt tgctgtttcc ttcagcctgc atggattttc tcatactttt 4260
tgaactgtaa tttttaagga agccaaattt gagggcagtt tgtcacagtt gatttccttc 4320
tctttccctt cgtcatgtga cctgatatcg ggggttagtt cgtcatcatt gatgagggtt 4380
gattatcaca gtttattact ctgaattggc tatccgcgtg tgtacctcta cctggagttt 4440
ttcccacggt ggatatttct tcttgcgctg agcgtaagag ctatctgaca gaacagttct 4500
tctttgcttc ctcgccagtt cgctcgctat gctcggttac acggctgcgg cgagcgctag 4560
tgataataag tgactgaggt atgtgctctt cttatctcct tttgtagtgt tgctcttatt 4620
ttaaacaact ttgcggtttt ttgatgactt tgcgattttg ttgttgcttt gcagtaaatt 4680
gcaagattta ataaaaaaac gcaaagcaat gattaaagga tgttcagaat gaaactcatg 4740
gaaacactta accagtgcat aaacgctggt catgaaatga cgaaggctat cgccattgca 4800
cagtttaatg atgacagccc ggaagcgagg aaaataaccc ggcgctggag aataggtgaa 4860
gcagcggatt tagttggggt ttcttctcag gctatcagag atgccgagaa agcagggcga 4920
ctaccgcacc cggatatgga aattcgagga cgggttgagc aacgtgttgg ttatacaatt 4980
gaacaaatta atcatatgcg tgatgtgttt ggtacgcgat tgcgacgtgc tgaagacgta 5040
tttccaccgg tgatcggggt tgctgcccat aaaggtggcg tttacaaaac ctcagtttct 5100
gttcatcttg ctcaggatct ggctctgaag gggctacgtg ttttgctcgt ggaaggtaac 5160
gacccccagg gaacagcctc aatgtatcac ggatgggtac cagatcttca tattcatgca 5220
gaagacactc tcctgccttt ctatcttggg gaaaaggacg atgtcactta tgcaataaag 5280
cccacttgct ggccggggct tgacattatt ccttcctgtc tggctctgca ccgtattgaa 5340
actgagttaa tgggcaaatt tgatgaaggt aaactgccca ccgatccaca cctgatgctc 5400
cgactggcca ttgaaactgt tgctcatgac tatgatgtca tagttattga cagcgcgcct 5460
aacctgggta tcggcacgat taatgtcgta tgtgctgctg atgtgctgat tgttcccacg 5520
cctgctgagt tgtttgacta cacctccgca ctgcagtttt tcgatatgct tcgtgatctg 5580
ctcaagaacg ttgatcttaa agggttcgag cctgatgtac gtattttgct taccaaatac 5640
agcaatagca atggctctca gtccccgtgg atggaggagc aaattcggga tgcctgggga 5700
agcatggttc taaaaaatgt tgtacgtgaa acggatgaag ttggtaaagg tcagatccgg 5760
atgagaactg tttttgaaca ggccattgat caacgctctt caactggtgc ctggagaaat 5820
gctctttcta tttgggaacc tgtctgcaat gaaattttcg atcgtctgat taaaccacgc 5880
tgggagatta gataatgaag cgtgcgcctg ttattccaaa acatacgctc aatactcaac 5940
cggttgaaga tacttcgtta tcgacaccag ctgccccgat ggtggattcg ttaattgcgc 6000
gcgtaggagt aatggctcgc ggtaatgcca ttactttgcc tgtatgtggt cgggatgtga 6060
agtttactct tgaagtgctc cggggtgata gtgttgagaa gacctctcgg gtatggtcag 6120
gtaatgaacg tgaccaggag ctgcttactg aggacgcact ggatgatctc atcccttctt 6180
ttctactgac tggtcaacag acaccggcgt tcggtcgaag agtatctggt gtcatagaaa 6240
ttgccgatgg gagtcgccgt cgtaaagctg ctgcacttac cgaaagtgat tatcgtgttc 6300

tggttggcga gctggatgat gagcagatgg ctgcattatc cagattgggt aacgattatc 6360
gcccaacaag tgcttatgaa cgtggtcagc gttatgcaag ccgattgcag aatgaatttg 6420
ctggaaatat ttctgcgctg gctgatgcgg aaaatatttc acgtaagatt attacccgct 6480
gtatcaacac cgccaaattg cctaaatcag ttgttgctct tttttctcac cccggtgaac 6540
tatctgcccg gtcaggtgat gcacttcaaa aagcctttac agataaagag gaattactta 6600
agcagcaggc atctaacctt catgagcaga aaaaagctgg ggtgatattt gaagctgaag 6660
aagttatcac tcttttaact tctgtgctta aaacgtcatc tgcatcaaga actagtttaa 6720
gctcacgaca tcagtttgct cctggagcga cagtattgta taagggcgat aaaatggtgc 6780
ttaacctgga caggtctcgt gttccaactg agtgtataga gaaaattgag gccattctta 6840
aggaacttga aaagccagca ccctgatgcg accacgtttt agtctacgtt tatctgtctt 6900
tacttaatgt cctttgttac aggccagaaa gcataactgg cctgaatatt ctctctgggc 6960
ccactgttcc acttgtatcg tcggtctgat aatcagactg ggaccacggt cccactcgta 7020
tcgtcggtct gattattagt ctgggaccac ggtcccactc gtatcgtcgg tctgattatt 7080
agtctgggac cacggtccca ctcgtatcgt cggtctgata atcagactgg gaccacggtc 7140
ccactcgtat cgtcggtctg attattagtc tgggaccatg gtcccactcg tatcgtcggt 7200
ctgattatta gtctgggacc acggtcccac tcgtatcgtc ggtctgatta ttagtctgga 7260
accacggtcc cactcgtatc gtcggtctga ttattagtct gggaccacgg tcccactcgt 7320
atcgtcggtc tgattattag tctgggacca cgatcccact cgtgttgtcg gtctgattat 7380
cggtctggga ccacggtccc acttgtattg tcgatcagac tatcagcgtg agactacgat 7440
tccatcaatg cctgtcaagg gcaagtattg acatgtcgtc gtaacctgta gaacggagta 7500
acctcggtgt gcggttgtat gcctgctgtg gattgctgct gtgtcctgct tatccacaac 7560
attttgcgca cggttatgtg gacaaaatac ctggttaccc aggccgtgcc ggcacgttaa 7620
ccgggctgca tccgatgcaa gtgtgtcgct gtcgacgagc tcgcgagctc ggacatgagg 7680
ttgccccgta ttcagtgtcg ctgatttgta ttgtctgaag ttgtttttac gttaagttga 7740
tgcagatcaa ttaatacgat acctgcgtca taattgatta tttgacgtgg tttgatggcc 7800
tccacgcacg ttgtgatatg tagatgataa tcattatcac tttacgggtc ctttccggtg 7860
atccgacagg ttacggggcg gcgacctcgc gggttttcgc tatttatgaa aattttccgg 7920
tttaaggcgt ttccgttctt cttcgtcata acttaatgtt tttatttaaa ataccctctg 7980
aaaagaaagg aaacgacagg tgctgaaagc gagctttttg gcctctgtcg tttcctttct 8040
ctgtttttgt ccgtggaatg aacaatggaa gtccgagctc atcgctaata acttcgtata 8100
gcatacatta tacgaagtta tattcgat 8128